# EUROPÄISCHE PATENTSCHRIFT

(11) **EP 3 992 760 B1**
(45) Veröffentlichungstag und Bekanntmachung des Hinweises auf die Patenterteilung: **25.09.2024**
(21) Anmeldenummer: 21166034.5
(22) Anmeldetag: 30.03.2021
(51) Int. Cl.: G06F 3/01, A61B 6/03, G06F 3/0488, G06F 3/041, G06F 3/0354, A61B 34/00, A61B 6/46, G06F 3/04847

(54) **VORRICHTUNG UND VERFAHREN ZUM BEDIENEN EINES MEDIZINISCHEN BILDGEBUNGSGERÄTS**
DEVICE AND METHOD FOR OPERATING A MEDICAL IMAGING DEVICE
DISPOSITIF ET PROCÉDÉ DE FONCTIONNEMENT D'UN APPAREIL D'IMAGERIE MÉDICALE

(43) Veröffentlichungstag der Anmeldung: 04.05.2022
(73) Patentinhaber: Siemens Healthineers AG, 91301 Forchheim (DE)
(72) Erfinder: Hamilton, Kathrin Friederike, 96103 Hallstadt (DE); Müller, Hans-Jürgen, 91362 Pretzfeld (DE); Schneider, Sebastian, 64850 Schaafheim (DE)
(74) Vertreter: Siemens Healthineers Patent Attorneys

(56) Entgegenhaltungen:
- EP-A1- 2 876 527
- US-A1- 2011 032 091
- US-A1- 2016 266 760
- US-A1- 2019 339 776

## Beschreibung

Die Erfindung betrifft eine Vorrichtung zum Bedienen eines medizinischen Bildgebungsgeräts, ein medizinisches Bildgebungsgerät und ein Verfahren zum Bedienen eines medizinischen Bildgebungsgeräts.

Beim Bedienen eines medizinischen Bildgebungsgeräts, beispielsweise eines Computertomographiegeräts, mittels einer Bedieneinheit, beispielsweise in Form einer Mensch-Maschine-Schnittstelle (Human-Machine-Interface, abgekürzt HMI), kann es zu einer Vielzahl unterschiedlicher Probleme kommen. Insbesondere betreffen diese Probleme die Sicherheit des medizinischen Bildgebungsgeräts und des Nutzers sowie die Ergonomie.

Gemäß einem grundlegenden Ablauf einer Mensch-Maschine-Interaktion ist vorgesehen, dass die Maschine dem Nutzer die verschiedenen Bedienoptionen signalisiert, der Nutzer, insbesondere mittels eines Inputs, eine Funktion auswählt und aktiviert, die Maschine die Eingabe registriert und verarbeitet, die Maschine die gewünschte Funktion aktiviert, die Maschine eine Rückmeldung (Feedback) für den Nutzer erzeugt und der Nutzer die Rückmeldung registriert und interpretiert. Missverständnisse oder Fehlinterpretationen innerhalb dieser Interaktionssequenz können an verschiedenen Punkten entstehen, wodurch die gesamte Interaktion negativ beeinflusst werden kann.

Mensch-Maschine-Schnittstellen an medizinischen Bildgebungsgeräten können beispielsweise visuelle, akustische und/oder haptische Rückmeldung für den Nutzer bereitstellen.

Visuelles Rückmeldung kann beispielsweise mittels einer Leuchtskala und/oder mittels eines Displays bereitgestellt werden. Akustische Rückmeldung kann beispielsweise mittels eines Signalgeber und/oder mittels eines Lautsprechers bereitgestellt werden. Haptische Rückmeldung kann beispielsweise mittels eines beweglichen Stellteils, mittels einer fühlbaren Oberflächeneigenschaft und/oder mittels eines Druccknopfs als Bedienelement bereitgestellt werden.

Eine von der Mensch-Maschine-Schnittstelle erzeugte Rückmeldung kann insbesondere auf ein bestimmtes Sinnesorgan des Nutzers abgestimmt sein. Da dieses Sinnesorgan nicht immer erreicht werden kann, kann eine erfolgreiche Rückmeldung somit ausbleiben. Beispielsweise wird eine visuelle Rückmeldung nur erkannt, wenn der Nutzer auf die entsprechende Anzeige schaut. Um die Chancen für eine erfolgreiche Rückmeldung zu erhöhen, kann die Rückmeldung auf mehreren verschiedenen Kanälen bereitgestellt werden, insbesondere redundant bereitgestellt werden. Beispielsweise kann vorgesehen sein, dass mittels einer Bedieneinheit sowohl visuelle Rückmeldung als auch akustische Rückmeldung bereitgestellt wird, insbesondere zeitlich parallel bereitgestellt wird.

Bewegliche Stellteile kann der Nutzer bei Betätigung erfühlen und somit eine haptische Rückmeldung erfassen. Um gute hygienische Verhältnisse zu erzeugen und die Entstehung von Fugen in der Oberfläche der Verkleidung des medizinischen Bildgebungsgeräts zu vermeiden, können kapazitive Bedienelemente verwendet werden. Diese können insbesondere mit einer glatten Oberfläche ausgeführt werden, um beispielsweise eine Berührung der glatten Oberfläche in Form einer Wischbewegung zu ermöglichen. Die glatte Oberfläche des kapazitiven Bedienelements kann zwar visuell gut erkennbar ausgestaltet sein, bei der Bedienung gibt es jedoch kein bewegliches Stellteil, das erfühlt werden kann, um dem Nutzer eine haptische Rückmeldung über den Erfolg seiner Eingabe bereitzustellen. Dies kann unterschiedliche unerwünschte Folgen haben, beispielsweise dass zusätzlich zu der Hand des Nutzers, welche die Berührung ausführt, wenigstens ein weiteres Sinnesorgan des Nutzers für das Bedienen des medizinischen Bildgebungsgeräts eingesetzt werden muss, um eine Rückmeldung einzuholen, beispielsweise visuell und/oder akustisch einzuholen. Dadurch kann sich das Risiko für das Ausbleiben einer erfolgreichen Rückmeldung erhöhen.

US 2011/032091 A1 offenbart ein Touchscreen-Gerät, umfassend eine Platte, Vibrationserzeugungsmittel, die auf einem Abschnitt der Platte montiert sind, und ein elastisches Element, das an der Platte befestigt ist.

EP 2 876 527 A1 offenbart eine Vorrichtung mit einem Berührungsbildschirm und einem haptischen Aktuator zum Bewegen des Berührungsbildschirms.

US 2016/266760 A1 offenbart ein Verfahren zum Planen einer medizinischen Bildgebungsuntersuchung eines Untersuchungsobjekts mittels eines medizinischen Bildgebungsgeräts, wobei eine Information auf einem Touch-Display haptisch dargeboten wird.

US 2019/339776 A1 offenbart ein System zum Erkennen und Reagieren auf Berührungseingaben mit haptischem Feedback.

Die Erfindung hat die Aufgabe, eine Vorrichtung zum Bedienen eines medizinischen Bildgebungsgeräts bereitzustellen, die auf einer Alternative zur herkömmlichen Anregung von Vibrationen eines Berührungsbereichs einer Bedieneinheit basiert.

Die Erfindung betrifft eine Vorrichtung zum Bedienen eines medizinischen Bildgebungsgeräts,
- wobei die Vorrichtung eine Bedieneinheit, eine Verbindungseinheit und eine Vibrationseinheit aufweist,
- wobei die Verbindungseinheit einen bedienelementseitigen Bereich, einen Randbereich und einen flexiblen Bereich aufweist, wobei der bedienelementseitige Bereich mittels des flexiblen Bereichs relativ zu dem Randbereich flexibel gelagert ist,
- wobei die Bedieneinheit einen Berührungsbereich aufweist, wobei der Berührungsbereich relativ zu dem bedienelementseitigen Bereich fixiert ist,
- wobei die Bedieneinheit dazu eingerichtet ist, ein Berührungssignal basierend auf einer Berührung des Berührungsbereichs zu generieren,
- wobei die Vibrationseinheit dazu eingerichtet ist, eine Vibration des Berührungsbereichs relativ zu dem Randbereich während der Berührung des Berührungsbereichs anzuregen, insbesondere derart anzuregen, dass die Vibration eine taktile Rückmeldung in Bezug auf die Berührung des Berührungsbereichs umfasst, insbesondere derart umfasst, dass die taktile Rückmeldung mittels der Berührung des Berührungsbereichs erfasst werden kann.

Insbesondere kann vorgesehen sein, dass die Vibrationseinheit dazu eingerichtet ist, eine Vibration des bedienelementseitigen Bereichs relativ zu dem Randbereich während der Berührung des Berührungsbereichs anzuregen, wobei die Vibration des bedienelementseitigen Bereichs relativ zu dem Randbereich während der Berührung des Berührungsbereichs die Vibration des Berührungsbereichs relativ zu dem Randbereich während der Berührung des Berührungsbereichs bewirkt, insbesondere dadurch bewirkt, dass der Berührungsbereich relativ zu dem bedienelementseitigen Bereich fixiert ist.

Für das Ertasten einer Position des Berührungsbereichs kann der Berührungsbereich und/oder der bedienelementseitige Bereich eine haptisch erfassbare Oberflächenstruktur aufweisen.

Erfindungsgemäß ist vorgesehen, dass die Vibrationseinheit einen Satz von Vibrationsmotoren aufweist. Als Vibrationsmotoren werden Motoren mit exzentrischer rotierender Masse (Eccentric Rotating Mass Motor, abgekürzt ERM-Motor) und/oder lineare Resonanzaktoren (Linear Resonant Actuators, abgekürzt LRA) verwendet.

Die Vibrationsmotoren können insbesondere in Scheibenform und/oder als Mikrovibrationsmotor ausgebildet sein. Insbesondere können Vibrationsmotoren verwendet werden, die dem Fachmann beispielsweise unter den englischen Bezeichnungen coin vibration motor, pager vibration motor oder pancake vibration motor bekannt sind.

Weiterhin kann vorgesehen sein, dass die Vibrationseinheit zum Anregen der Vibration beispielsweise wenigstens einen akustischen Signalgeber und/oder wenigstens einen Piezo-Aktor aufweist.

Eine Ausführungsform sieht vor, dass die Bedieneinheit ferner dazu eingerichtet ist, das Berührungssignal an eine Datenverarbeitungseinheit zu senden,
- wobei die Datenverarbeitungseinheit dazu eingerichtet ist, das Berührungssignal zu empfangen, basierend auf dem Berührungssignal ein Vibrationssignal zu generieren und das Vibrationssignal an die Vibrationseinheit zu senden,
- wobei die Vibrationseinheit ferner dazu eingerichtet ist, das Vibrationssignal zu empfangen und die Vibration des Berührungsbereichs relativ zu dem Randbereich basierend auf dem Vibrationssignal anzuregen.

Eine Ausführungsform sieht vor, dass die Vorrichtung ferner die Datenverarbeitungseinheit aufweist.

Eine Ausführungsform sieht vor, dass die Berührung des Berührungsbereichs eine Eingabe eines Werts für das Bedienen des medizinischen Bildgebungsgeräts betrifft, insbesondere bewirkt, wobei die Vibration des Berührungsbereichs relativ zu dem Randbereich während der Berührung des Berührungsbereichs eine taktile Rückmeldung, welche den Wert für das Bedienen des medizinischen Bildgebungsgeräts betrifft, umfasst.

Eine Ausführungsform sieht vor, dass die Vibration des Berührungsbereichs relativ zu dem Randbereich während der Berührung des Berührungsbereichs ein Trägersignal bildet, wobei das Trägersignal mit einem Nutzsignal, welches den Wert für das Bedienen des medizinischen Bildgebungsgeräts betrifft, moduliert ist.

Weiterhin kann vorgesehen sein, dass ein Parameter der Vibration von dem Wert für das Bedienen des medizinischen Bildgebungsgeräts abhängig ist, insbesondere monoton abhängig ist.

Der Parameter der Vibration kann beispielsweise aus der Vibrationsparameter-Gruppe gewählt sein, welche aus einer Dauer der Vibration, einer Amplitude der Vibration, einer Frequenz der Vibration und Kombinationen davon besteht.

Weiterhin kann vorgesehen sein, dass der Wert für das Bedienen des medizinischen Bildgebungsgeräts von einem Parameter der Berührung des Berührungsbereichs abhängig ist, insbesondere monoton abhängig ist. Der Parameter der Berührung des Berührungsbereichs kann beispielsweise ein Parameter einer Wischbewegung der Berührung des Berührungsbereichs sein. Der Parameter der Berührung des Berührungsbereichs kann beispielsweise aus der Berührungsparameter-Gruppe gewählt sein, welche aus einer Dauer der Berührung, einer Intensität der Berührung, einer Geschwindigkeit der Wischbewegung der Berührung, einem Pfad der Wischbewegung der Berührung, einer von der Wischbewegung zurückgelegten Strecke und Kombinationen davon besteht.

Beispielsweise kann bei einem ein kapazitiven Schieberegler, welcher über kein bewegliches Stellteil verfügt, eine zunehmende Vibrationsstärke Rückmeldung über die Schalterstellung geben. Dadurch kann beispielsweise eine Lageänderung eines beweglichen Stellteils eines Schiebereglers imitiert werden.

Die Berührung des Berührungsbereichs kann beispielsweise eine Wischbewegung entlang des Berührungsbereichs umfassen. Insbesondere kann vorgesehen sein, dass entlang des Pfades der Wischbewegung eine Mehrzahl von Positionen eines Fingers und/oder eines Zeigers, der für die Berührung des Berührungsbereichs verwendet wird, relativ zu dem Berührungsbereich vorgesehen sind. Insbesondere kann jeder Position der Mehrzahl von Positionen ein entsprechender Wert für das Bedienen des medizinischen Bildgebungsgeräts zugeordnet sein. Die Vibration des Berührungsbereichs relativ zu dem Randbereich kann beispielsweise eine taktile Rückmeldung dazu umfassen, an welcher Position der Mehrzahl von Positionen sich der Finger und/oder eines Zeiger befindet.

Erfindungsgemäß ist vorgesehen, dass die Vibrationsmotoren des Satzes von Vibrationsmotoren derart relativ zueinander angeordnet sind, dass eine Referenzebene durch jeden Vibrationsmotor des Satzes von Vibrationsmotoren verläuft.

Erfindungsgemäß ist vorgesehen, dass die Vibrationsmotoren des Satzes von Vibrationsmotoren derart um eine zu der Referenzebene senkrechte Referenzachse herum angeordnet sind, dass der Satz von Vibrationsmotoren für jede beliebige Halbgerade, die von der Referenzachse ausgeht und in der Referenzebene liegt, einen entsprechenden Vibrationsmotor aufweist, sodass ein auf die Referenzachse bezogener Winkelabstand zwischen dieser Halbgeraden und der Motorachse des entsprechenden Vibrationsmotors, nicht größer als 60 Grad ist, insbesondere nicht größer als 45 Grad ist, beispielsweise nicht größer als 30 Grad ist.

Insbesondere kann vorgesehen sein, dass die Referenzachse durch einen zentralen Bereich des bedienelementseitigen Bereichs verläuft und/oder dass sich der bedienelementseitige Bereich flächig im Wesentlichen parallel zu der Referenzebene erstreckt.

Insbesondere kann vorgesehen sein, dass ein Abstand zwischen der Referenzachse und dem Berührungsbereich kleiner ist als ein Abstand zwischen der Referenzachse und demjenigen Vibrationsmotor des Satzes von Vibrationsmotoren, welcher der Referenzachse am nächsten ist.

Erfindungsgemäß ist vorgesehen, dass für jeden Vibrationsmotor des Satzes von Vibrationsmotoren eine Motorachse dieses Vibrationsmotors in der Referenzebene liegt und eine Rotationsachse eines diesen Vibrationsmotor bildenden Motors mit exzentrischer rotierender Masse, um welche die exzentrische Masse rotiert, oder eine Linearachse eines diesen Vibrationsmotor bildenden linearen Resonanzaktors ist.

Weiterhin kann vorgesehen sein, dass die Vibrationseinheit wenigstens einen weiteren Vibrationsmotor aufweist, wobei der wenigstens eine weitere Vibrationsmotor nicht in dem Satz von Vibrationsmotoren enthalten ist.

Weiterhin kann vorgesehen sein, dass die Verbindungseinheit einen flexiblen Dichtungsring aufweist, wobei eine erste ringförmige Kontaktfläche des flexiblen Dichtungsrings relativ zu dem bedienelementseitigen Bereich fixiert ist. Insbesondere kann vorgesehen sein, dass eine zweite ringförmige Kontaktfläche des flexiblen Dichtungsrings wenigstens einen Teilbereich des Randbereichs bildet. Insbesondere kann vorgesehen sein, dass ein ringförmiger Teilbereich des flexiblen Dichtungsrings wenigstens einen Teilbereich des flexiblen Bereichs bildet.

Der ringförmige Teilbereich des flexiblen Dichtungsrings, der den wenigstens einen Teilbereich des flexiblen Bereichs bildet, kann sich insbesondere zwischen der ersten ringförmigen Kontaktfläche des flexiblen Dichtungsrings und der zweiten ringförmigen Kontaktfläche des flexiblen Dichtungsrings befinden.

Die Erfindung betrifft ferner ein medizinisches Bildgebungsgerät, aufweisend die erfindungsgemäße Vorrichtung und eine Verkleidung zur Abgrenzung eines Innenbereichs des medizinischen Bildgebungsgeräts gegen eine Umgebung, wobei die Umgebung das medizinische Bildgebungsgerät umgibt, wobei der Randbereich an der Verkleidung relativ zu der Verkleidung fixiert ist, insbesondere derart fixiert ist, dass der bedienelementseitige Bereich mittels des flexiblen Bereichs relativ zu der Verkleidung flexibel gelagert ist.

Eine Ausführungsform sieht vor, dass die Verkleidung eine Aussparung aufweist, in welche zumindest ein Teil der Vorrichtung aufnehmbar ist, wobei der zumindest eine Teil der Vorrichtung in die Aussparung aufgenommen ist, wobei der Randbereich entlang wenigstens einer ringförmigen Kontur, welche die Aussparung umgibt, formschlüssig und/oder flüssigkeitsundurchlässig an die Verkleidung anschließt.

Die Erfindung betrifft ferner ein Verfahren zum Bedienen eines medizinischen Bildgebungsgeräts mittels einer Vorrichtung,
- wobei die Vorrichtung eine Bedieneinheit, eine Verbindungseinheit und eine Vibrationseinheit aufweist,
- wobei die Verbindungseinheit einen bedienelementseitigen Bereich, einen Randbereich und einen flexiblen Bereich aufweist, wobei der bedienelementseitige Bereich mittels des flexiblen Bereichs relativ zu dem Randbereich flexibel gelagert ist,
- wobei die Bedieneinheit einen Berührungsbereich aufweist, wobei der Berührungsbereich relativ zu dem bedienelementseitigen Bereich fixiert ist,
- wobei mittels der Bedieneinheit ein Berührungssignal basierend auf einer Berührung des Berührungsbereichs generiert wird,
- wobei mittels der Vibrationseinheit eine Vibration des Berührungsbereichs relativ zu dem Randbereich während der Berührung des Berührungsbereichs angeregt wird, insbesondere derart angeregt wird, dass die Vibration eine taktile Rückmeldung in Bezug auf die Berührung des Berührungsbereichs umfasst, insbesondere derart umfasst, dass die taktile Rückmeldung mittels der Berührung des Berührungsbereichs erfasst wird.

Insbesondere kann vorgesehen sein, dass mittels der Vibrationseinheit die Vibration des Berührungsbereichs relativ zu dem Randbereich während der Berührung des Berührungsbereichs angeregt wird, indem mittels der Vibrationseinheit eine Vibration des bedienelementseitigen Bereichs relativ zu dem Randbereich während der Berührung des Berührungsbereichs angeregt wird und durch die Vibration des bedienelementseitigen Bereichs relativ zu dem Randbereich während der Berührung des Berührungsbereichs die Vibration des Berührungsbereichs relativ zu dem Randbereich während der Berührung des Berührungsbereichs bewirkt wird, insbesondere dadurch bewirkt wird, dass der Berührungsbereich relativ zu dem bedienelementseitigen Bereich fixiert ist.

Insbesondere kann das medizinische Bildgebungsgerät bedient werden, indem durch die Berührung des Berührungsbereichs eine Eingabe eines Werts für das Bedienen des medizinischen Bildgebungsgeräts bewirkt wird.

Eine Ausführungsform sieht vor, dass mittels der Bedieneinheit das Berührungssignal an eine Datenverarbeitungseinheit gesendet wird, wobei mittels der Datenverarbeitungseinheit das Berührungssignal empfangen wird, basierend auf dem Berührungssignal ein Vibrationssignal generiert wird und das Vibrationssignal an die Vibrationseinheit gesendet wird, wobei mittels der Vibrationseinheit das Vibrationssignal empfangen wird und basierend auf dem Vibrationssignal die Vibration des Berührungsbereichs relativ zu dem Randbereich anzuregen.

Eine Ausführungsform sieht vor, dass die Berührung des Berührungsbereichs eine Eingabe eines Werts für das Bedienen des medizinischen Bildgebungsgeräts betrifft, insbesondere bewirkt, wobei die Vibration des Berührungsbereichs relativ zu dem Randbereich während der Berührung des Berührungsbereichs eine taktile Rückmeldung, welche den Wert für das Bedienen des medizinischen Bildgebungsgeräts betrifft, umfasst.

Eine Ausführungsform sieht vor, dass die Vibration des Berührungsbereichs relativ zu dem Randbereich während der Berührung des Berührungsbereichs ein Trägersignal bildet, wobei das Trägersignal mit einem Nutzsignal, welches den Wert für das Bedienen des medizinischen Bildgebungsgeräts betrifft, moduliert wird.

Eine Ausführungsform sieht vor, dass ein Parameter der Vibration von dem Wert für das Bedienen des medizinischen Bildgebungsgeräts monoton abhängig ist.

Weiterhin kann vorgesehen sein, dass die Vorrichtung zusätzlich zu der taktilen Rückmeldung beispielsweise visuelle, akustische und/oder haptische Rückmeldung bereitstellen kann, die insbesondere den Wert für das Bedienen des medizinischen Bildgebungsgeräts betreffen kann. Dazu können beispielsweise Leuchtskalen, Displays, Tongeber und/oder eine in bestimmten Bereichen veränderte Oberflächenstruktur des Berührungsbereichs verwendet werden. Durch den Zusammenschluss dieser unterschiedlicher Rückmeldungsarten wird die Bedienergonomie und Bediensicherheit zusätzlich verbessert.

Dem Nutzer kann somit auf mehrere Arten eine erfolgreich registrierte Bedienung des medizinischen Bildgebungsgeräts signalisiert werden. Durch ein Ausbleiben jeglicher Rückmeldung kann dem Nutzer eine nicht erfolgreiche Bedienung signalisiert werden. Die Berührung kann beispielsweise durch einen Nutzer erfolgen, der sich in der Umgebung befindet. Als Nutzer kommen beispielsweise medizinisch-technisches Personal, Radiologen und/oder Servicemitarbeiter in Betracht.

Je eindeutiger dem Nutzer erfolgreiche Eingabe einerseits und nicht erfolgreiche Eingaben andererseits signalisiert werden, desto geringer ist die Wahrscheinlichkeit, dass der Nutzer das Element erneut bedient, um eine Aktivierung sicherzustellen. Eine erneute Bedienung kann zu negativen Konsequenzen führen, welche Nutzer und System beeinträchtigen können. Wenn das System eine gewisse Zeit für eine Systemantwort benötigt, ist es möglich, dass der Nutzer diese Zeit nicht als Wartezeit, sondern als nicht erfolgreiche Eingabe erkennt. Dadurch ist es möglich, dass der Nutzer eine erneute Eingabe tätigt, welche in unvorhersehbarer Weise mit der vorherigen Eingabe interferiert. Dadurch, dass die Vibration des Berührungsbereichs relativ zu dem Randbereich während der Berührung des Berührungsbereichs angeregt wird, kann dem Nutzer während der Berührung signalisiert werden, ob die Eingabe erfolgreich oder nicht erfolgreich ist, sodass unnötige erneute Eingaben vermieden werden können.

Indem dem Nutzer eine optimale Bedienbarkeit ermöglicht wird, können Bedienvorgänge reibungsloser und effektivier ausgeführt werden. Die Erhöhung von Bediengenauigkeit und Bediensicherheit kann die Zahl von Fehlbedienungen, die in Personen- oder Geräteschäden resultieren könnten, reduziert werden.

Das medizinische Bildgebungsgerät kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Szintigraphiegerät, einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem SPECT-CT-Gerät, besteht. Das medizinische Bildgebungsgerät kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Das medizinische Bildgebungsgerät kann beispielsweise eine Gantry mit einer tunnelförmigen Öffnung aufweisen. Insbesondere kann in die tunnelförmige Öffnung ein Patient eingeführt werden, um mittels des medizinischen Bildgebungsgeräts untersucht zu werden.

Das medizinische Bildgebungsgerät kann insbesondere die Datenverarbeitungseinheit aufweisen. Insbesondere kann vorgesehen sein, dass die Datenverarbeitungseinheit mittels einer Datenübertragungsverbindung, insbesondere mittels einer bidirektionalen Datenübertragungsverbindung, mit der Vorrichtung verbunden ist und/oder dass die Datenverarbeitungseinheit mittels elektrischer Leitungen zur Stromübertragung und/oder Spannungsübertragung mit der Vorrichtung verbunden ist. Die Datenverarbeitungseinheit kann sich beispielsweise innerhalb des Innenbereichs und/oder außerhalb der Gantry, insbesondere in einer von der Gantry separaten Recheneinheit, befinden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
Die Fig. 1 zeigt ein medizinisches Bildgebungsgerät mit einer Vorrichtung zum Bedienen des medizinischen Bildgebungsgeräts.
Die Fig. 2 zeigt einen Bereich der Verkleidung des medizinischen Bildgebungsgeräts mit der Vorrichtung zum Bedienen des medizinischen Bildgebungsgeräts.
Die Fig. 3 zeigt eine vereinfachte Darstellung der Vorrichtung zum Bedienen des medizinischen Bildgebungsgeräts.
Die Fig. 4 zeigt die Vorrichtung zum Bedienen des medizinischen Bildgebungsgeräts in einer ersten Ansicht.
Die Fig. 5 zeigt die Vorrichtung zum Bedienen des medizinischen Bildgebungsgeräts in einer zweiten Ansicht.
Die Fig. 6 zeigt eine Anordnung von Komponenten der Vorrichtung zum Bedienen des medizinischen Bildgebungsgeräts.
Die Fig. 7 zeigt einen Teil der Vorrichtung zum Bedienen des medizinischen Bildgebungsgeräts.
Die Fig. 8 zeigt eine schichtförmige Anordnung von Teileinheiten der Vorrichtung zum Bedienen des medizinischen Bildgebungsgeräts.
Die Fig. 9 zeigt eine Vibrationseinheit mit einem Satz von Vibrationsmotoren.
Die Fig. 10 zeigt eine Leuchteinheit.
Die Fig. 11 zeigt eine Sensoreinheit.
Die Fig. 12 zeigt eine Deckschicht.
Die Fig. 13 zeigt eine Schnittdarstellung durch die Vorrichtung zum Bedienen des medizinischen Bildgebungsgeräts.
Die Fig. 14 zeigt ein Ablaufdiagramm für ein Verfahren zum Bedienen eines medizinischen Bildgebungsgeräts.
Die Fig. 15 zeigt ein weiteres Beispiel für ein Ablaufdiagramm für ein Verfahren zum Bedienen eines medizinischen Bildgebungsgeräts.

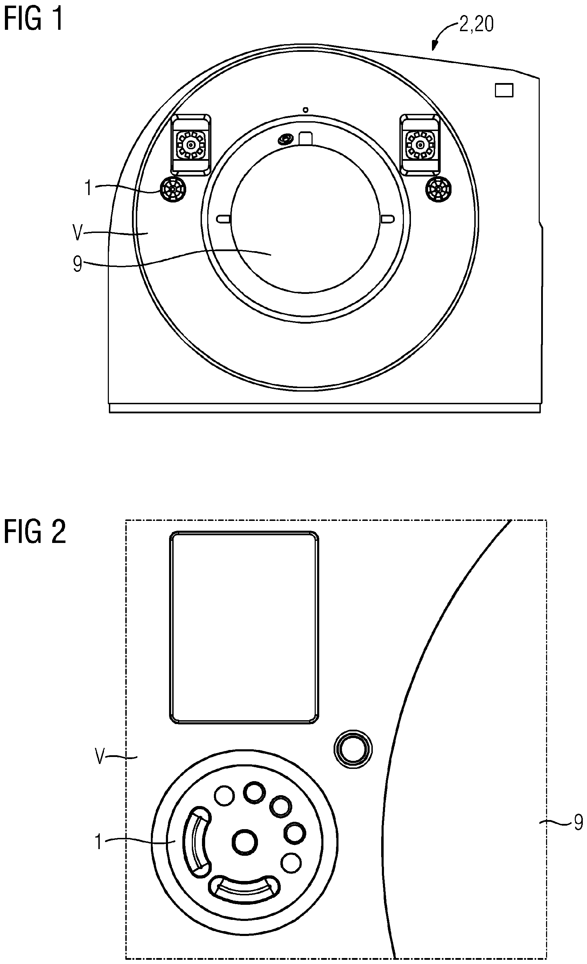

Die Fig. 1 zeigt ein medizinisches Bildgebungsgerät 2 mit einer Vorrichtung 1 zum Bedienen des medizinischen Bildgebungsgeräts 2 und eine Verkleidung V zur Abgrenzung eines Innenbereichs 4 des medizinischen Bildgebungsgeräts 2 gegen eine Umgebung 5, wobei die Umgebung 5 das medizinische Bildgebungsgerät 2 umgibt.

Das medizinische Bildgebungsgerät 2 weist eine Gantry 20 mit einer tunnelförmigen Öffnung 9 auf. Insbesondere kann in die tunnelförmige Öffnung 9 ein Patient eingeführt werden, um mittels des medizinischen Bildgebungsgeräts 2 untersucht zu werden.

Die Fig. 2 zeigt einen Bereich der Verkleidung V des medizinischen Bildgebungsgeräts 2 mit der Vorrichtung 1 zum Bedienen des medizinischen Bildgebungsgeräts 2.

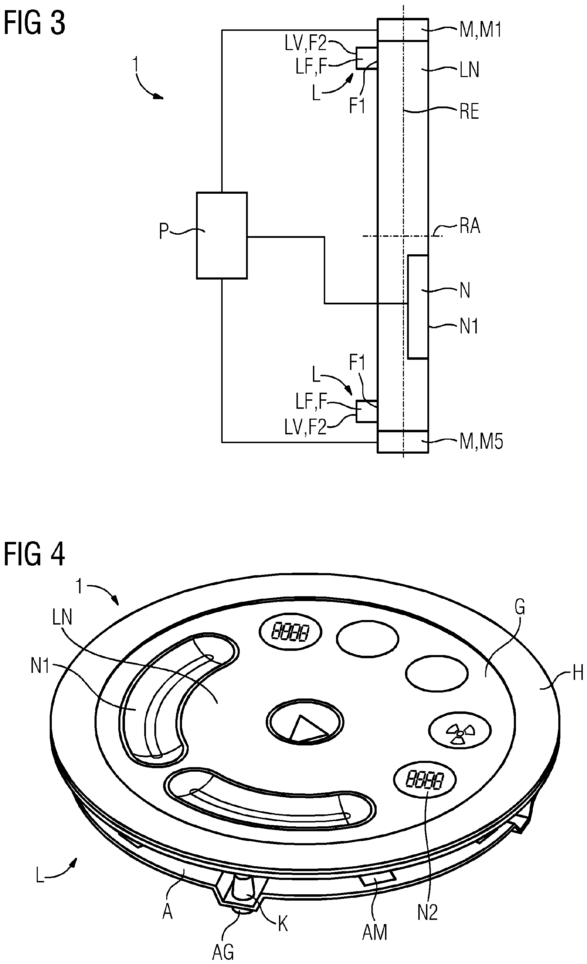

Die Fig. 3 zeigt eine vereinfachte Schnittdarstellung der Vorrichtung 1 zum Bedienen des medizinischen Bildgebungsgeräts 2. Die Vorrichtung 1 weist eine Bedieneinheit N, eine Verbindungseinheit L und eine Vibrationseinheit M auf. Die Verbindungseinheit L weist einen bedienelementseitigen Bereich LN, einen Randbereich LV und einen flexiblen Bereich LF auf, wobei der bedienelementseitige Bereich LN mittels des flexiblen Bereichs LF relativ zu dem Randbereich LV flexibel gelagert ist, wobei die Bedieneinheit N einen Berührungsbereich N1 aufweist, wobei der Berührungsbereich N1 relativ zu dem bedienelementseitigen Bereich LN fixiert ist, wobei die Bedieneinheit N dazu eingerichtet ist, ein Berührungssignal basierend auf einer Berührung des Berührungsbereichs N1 zu generieren S1.

Die Vibrationseinheit M ist dazu eingerichtet, eine Vibration des Berührungsbereichs N1 relativ zu dem Randbereich LV während der Berührung des Berührungsbereichs N1 anzuregen S2, insbesondere derart anzuregen, dass die Vibration eine taktile Rückmeldung in Bezug auf die Berührung des Berührungsbereichs N1 umfasst, insbesondere derart umfasst, dass die taktile Rückmeldung mittels der Berührung des Berührungsbereichs N1 erfasst werden kann.

Die Vorrichtung 1 weist ferner die Datenverarbeitungseinheit P auf. Die Bedieneinheit N ist ferner dazu eingerichtet, das Berührungssignal an eine Datenverarbeitungseinheit P zu senden S11, wobei die Datenverarbeitungseinheit P dazu eingerichtet ist, das Berührungssignal zu empfangen SP0, basierend auf dem Berührungssignal ein Vibrationssignal zu generieren SP und das Vibrationssignal an die Vibrationseinheit M zu senden SP1, wobei die Vibrationseinheit M ferner dazu eingerichtet ist, das Vibrationssignal zu empfangen S20 und die Vibration des Berührungsbereichs N1 relativ zu dem Randbereich LV basierend auf dem Vibrationssignal anzuregen S2.

Die Fig. 4 zeigt die Vorrichtung 1 zum Bedienen des medizinischen Bildgebungsgeräts 2 in einer ersten Ansicht. Die Berührung des Berührungsbereichs N1 betrifft, insbesondere bewirkt, eine Eingabe eines Werts für das Bedienen des medizinischen Bildgebungsgeräts 2, wobei die Vibration des Berührungsbereichs N1 relativ zu dem Randbereich LV während der Berührung des Berührungsbereichs N1 eine taktile Rückmeldung, welche den Wert für das Bedienen des medizinischen Bildgebungsgeräts 2 betrifft, umfasst.

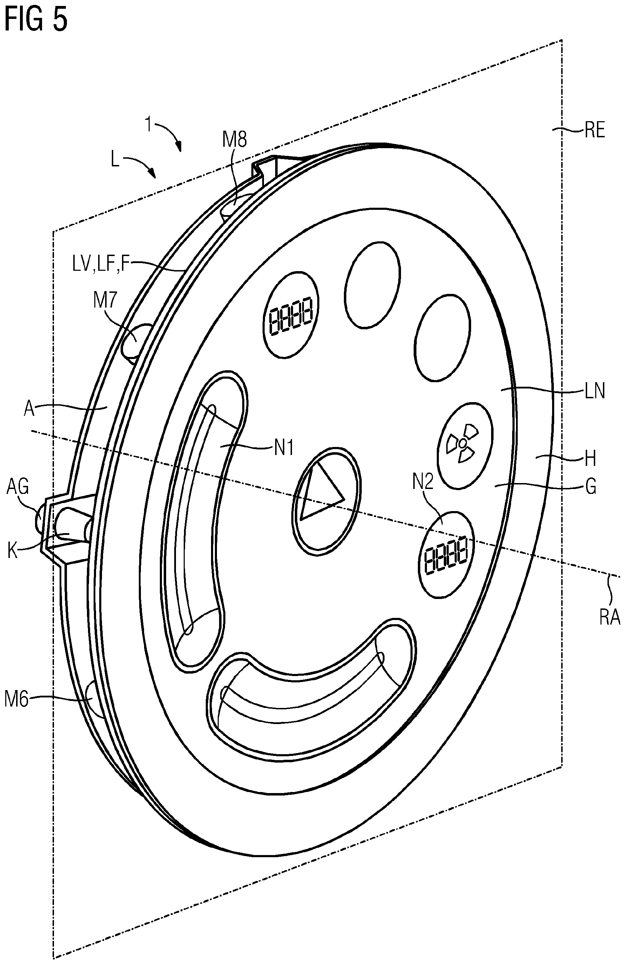

Die Fig. 5 zeigt die Vorrichtung 1 zum Bedienen des medizinischen Bildgebungsgeräts 2 in einer zweiten Ansicht. Die Referenzachse RA verläuft durch einen zentralen Bereich des bedienelementseitigen Bereichs LN. Der bedienelementseitige Bereich LN erstreckt sich flächig im Wesentlichen parallel zu der Referenzebene RE

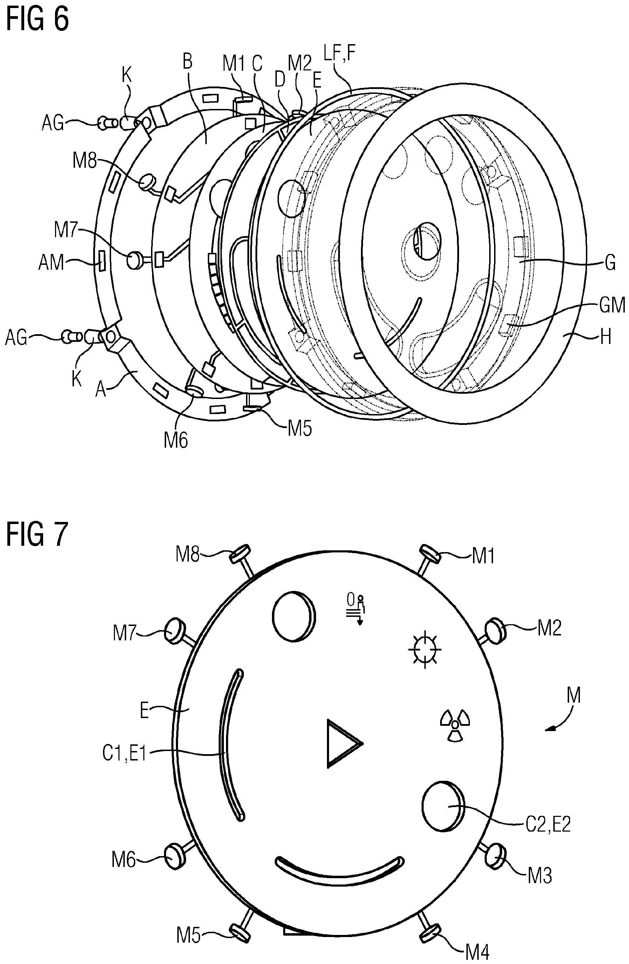

Die Fig. 6 zeigt eine Anordnung von Komponenten der Vorrichtung 1 zum Bedienen des medizinischen Bildgebungsgeräts 2. Das Gehäuse G weist einen Satz von Aussparungen GM auf. Für jeden Vibrationsmotor des Satzes von Vibrationsmotoren M1-M8 ist eine Aussparung des Satzes von Aussparungen GM vorgesehen, in welche dieser Vibrationsmotor formschlüssig aufgenommen werden kann. Zusätzlich kann der in die Aussparung aufgenommene Vibrationsmotor mit dem Gehäuse G verklebt sein.

Der Lagerungsring A weist einen Satz von Öffnungen AM auf. Für jeden Vibrationsmotor des Satzes von Vibrationsmotoren M1-M8 ist eine Öffnung des Satzes von Öffnungen AM vorgesehen, durch welche dieser Vibrationsmotor durchgesteckt werden kann, um in die entsprechende Aussparung aufgenommen zu werden, wenn der Lagerungsring A mit dem Gehäuse G verbunden ist.

Die Fig. 7 zeigt einen Teil der Vorrichtung 1 zum Bedienen des medizinischen Bildgebungsgeräts 2.

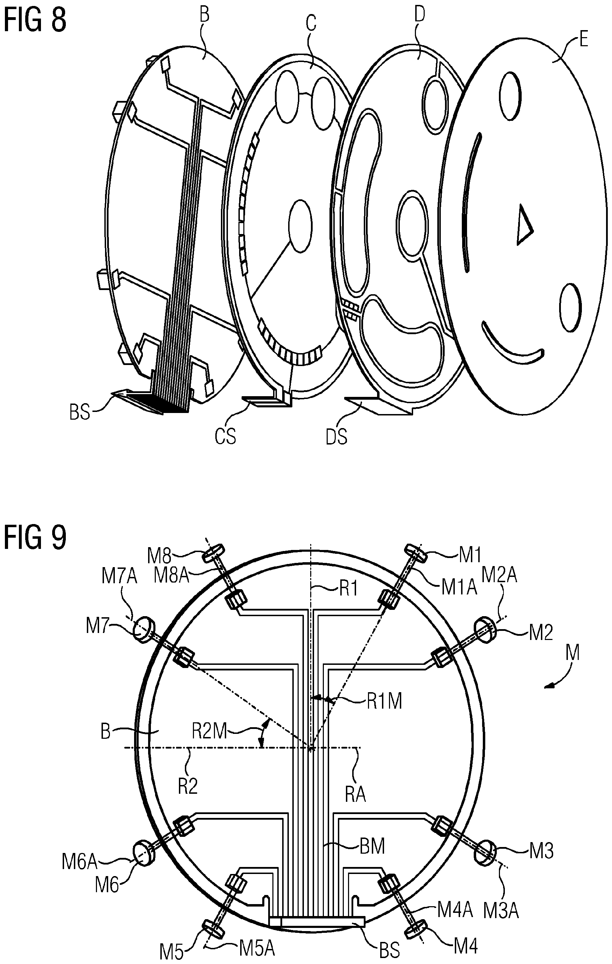

Die Fig. 8 zeigt eine schichtförmige Anordnung von Teileinheiten der Vorrichtung 1 zum Bedienen des medizinischen Bildgebungsgeräts 2.

Die Vorrichtung 1 weist die Elektronik-Trageinheit B der Vibrationseinheit M, die Leuchteinheit C, die Sensoreinheit D und die Deckschicht E auf.

Die Fig. 9 zeigt die Vibrationseinheit M mit einem Satz von Vibrationsmotoren M1, M2, M3, M4, M5, M6, M7 und M8. Die Vibrationsmotoren des Satzes von Vibrationsmotoren M1-M8 sind derart relativ zueinander angeordnet, dass eine Referenzebene RE durch jeden Vibrationsmotor des Satzes von Vibrationsmotoren M1-M8 verläuft, wobei die Vibrationsmotoren des Satzes von Vibrationsmotoren M1-M8 derart um eine zu der Referenzebene RE senkrechte Referenzachse RA herum angeordnet sind, dass der Satz von Vibrationsmotoren M1-M8 für jede beliebige Halbgerade R1, R2, die von der Referenzachse RA ausgeht und in der Referenzebene RE liegt, einen entsprechenden Vibrationsmotor aufweist, sodass ein auf die Referenzachse RA bezogener Winkelabstand R1M, R2M zwischen dieser Halbgeraden R1, R2 und dem entsprechenden Vibrationsmotor, insbesondere zwischen dieser Halbgeraden R1, R2 und der Motorachse des entsprechenden Vibrationsmotors nicht größer als 45 Grad ist.

Jede der Motorachsen M1A, M2A, M3A, M4A, M5A, M6A, M7A und M8A liegt in der Referenzebene RE und ist senkrecht zu der Referenzachse RA. Die Vibrationseinheit M weist ferner die Elektronik-Trageinheit B mit elektrischen Leitungen BM zur Ansteuerung der Vibrationsmotoren des Satzes von Vibrationsmotoren M1-M8 auf. Die Elektronik-Trageinheit B weist eine Schnittstelle BS für eine Datenübertragungsverbindung mit der Datenverarbeitungseinheit P auf.

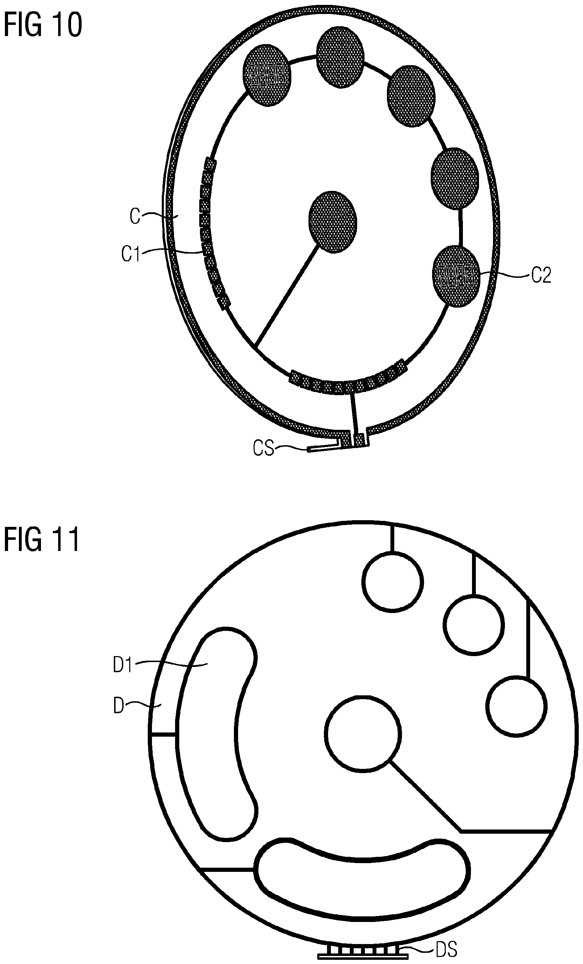

Die Fig. 10 zeigt die Leuchteinheit C. Die Leuchteinheit C ist dazu eingerichtet, ein Leuchten des Berührungsbereichs N1 während der Berührung des Berührungsbereichs anzuregen, insbesondere derart anzuregen, dass das Leuchten eine visuelle Rückmeldung in Bezug auf die Berührung des Berührungsbereichs umfasst, insbesondere derart umfasst. Die visuelle Rückmeldung kann insbesondere den Wert für das Bedienen des medizinischen Bildgebungsgeräts betreffen. Die Leuchteinheit C kann beispielsweise zum Anregen des Leuchtens eine Elektrolumineszenzfolie aufweisen.

Die Leuchteinheit C weist an dem Bereich des Berührungsbereichs N1 eine Leuchtskala C1 auf. Die Leuchteinheit C weist an dem Bereich des Anzeigeelements N2 ein Display C2 auf. Die Leuchteinheit C weist eine Schnittstelle CS für eine Datenübertragungsverbindung mit der Datenverarbeitungseinheit P auf.

Die Fig. 11 zeigt die Sensoreinheit D. Die Sensoreinheit D weist zum Generieren des Berührungssignals basierend auf einer Berührung des Berührungsbereichs N1 das kapazitive Sensorelement D1 auf. Die Sensoreinheit D weist eine Schnittstelle DS für eine Datenübertragungsverbindung mit der Datenverarbeitungseinheit P auf.

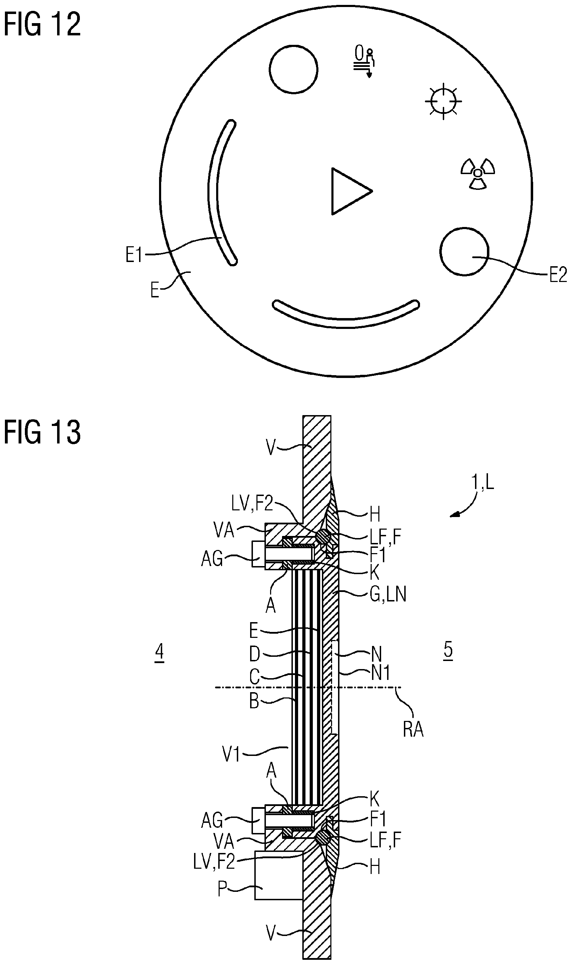

Die Fig. 12 zeigt die Deckschicht E. Die Deckschicht E weist eine Deckfolie mit der Öffnung E1 zur visuellen Begrenzung der darunterliegenden Leuchtskala C1 und der Öffnung E2 zur visuellen Begrenzung des darunterliegenden Displays C2 auf. Die Deckschicht E weist weitere Öffnungen in Form von Symbolen für weitere Bedienelemente und/oder Anzeigeelemente auf.

Die Fig. 13 zeigt eine Schnittdarstellung der Vorrichtung 1 zum Bedienen des medizinischen Bildgebungsgeräts 2.

Die Verbindungseinheit L weist ferner das Gehäuse G, die Gewindeeinsätze K und den Abschlussring H auf. Das Gehäuse G bildet den bedienelementseitigen Bereich LN. Die Schrauben AG sind durch Löcher in dem Verbindungsbereich VA der Verkleidung und durch Löcher in dem Lagerungsring A durchgeführt und mit den Gewindeeinsätzen K verschraubt. Die Gewindeeinsätze K können beispielsweise mit den Gehäuse G verklebt und/oder verschmolzen sein.

Der Abschlussring H weist eine Dichtlippe auf, die an die Verkleidung V anschließt, insbesondere formschlüssig und flüssigkeitsundurchlässig and die Verkleidung anschließt. Die Kontaktfläche des Abschlussrings H, die an die Verkleidung V anschließt, kann beispielsweise alternativ oder zusätzlich zu F2 wenigstens einen Teilbereich des Randbereichs LV bilden. Ein ringförmiger Teilbereich des Abschlussrings H kann insbesondere wenigstens einen Teilbereich des flexiblen Bereichs LF bilden. Dazu kann der Abschlussring H aus einem flexiblen Material hergestellt sein. Die Kontaktfläche des Abschlussrings H, die an den bedienelementseitigen Bereich LN anschließt, ist relativ zu dem bedienelementseitigen Bereich LN fixiert. Der Abschlussring H kann beispielsweise mittels einer stoffschlüssigen Verbindung, insbesondere mittels einer Schmelzverbindung und/oder mittels einer Mehrkomponenten-Spritzguss-Verbindung mit dem Gehäuse G verbunden sein.

Die Kontaktfläche des Lagerungsrings A, die an den Verbindungsbereich VA der Verkleidung V anschließt, kann beispielsweise zusätzlich zu F2 und/oder zusätzlich zu der Kontaktfläche des Abschlussrings H, die an die Verkleidung V anschließt, wenigstens einen Teilbereich des Randbereichs LV bilden. Ein ringförmiger Teilbereich des Lagerungsrings A kann insbesondere wenigstens einen Teilbereich des flexiblen Bereichs LF bilden. Dazu kann der Lagerungsrings A aus einem flexiblen Material hergestellt sein. Die Kontaktfläche des Lagerungsrings A, die an den bedienelementseitigen Bereich LN anschließt, ist relativ zu dem bedienelementseitigen Bereich LN fixiert. Der Lagerungsring A kann beispielsweise mittels einer stoffschlüssigen Verbindung, insbesondere mittels einer Schmelzverbindung und/oder mittels einer Mehrkomponenten-Spritzguss-Verbindung mit dem Gehäuse G verbunden sein.

Der Randbereich LV kann insbesondere einen von dem Lagerungsring A gebildeten Teilbereich des Randbereichs LV und/oder einen von dem Abschlussring H gebildeten Teilbereich des Randbereichs LV umfassen. Der flexible Bereich LF kann insbesondere einen von dem Lagerungsring A gebildeten Teilbereich des flexiblen Bereichs LF und/oder einen von dem Abschlussring H gebildeten Teilbereich des flexiblen Bereichs LF umfassen.

Die Verbindungseinheit L weist einen flexiblen Dichtungsring F auf, wobei eine erste ringförmige Kontaktfläche F1 des flexiblen Dichtungsrings F relativ zu dem bedienelementseitigen Bereich LN fixiert ist, wobei eine zweite ringförmige Kontaktfläche F2 des flexiblen Dichtungsrings F wenigstens einen Teilbereich des Randbereichs LV bildet, wobei ein ringförmiger Teilbereich des flexiblen Dichtungsrings F wenigstens einen Teilbereich des flexiblen Bereichs LF bildet.

Die Verkleidung V weist eine Aussparung V1 auf, in welche zumindest ein Teil der Vorrichtung 1 aufnehmbar ist, wobei der zumindest eine Teil der Vorrichtung 1 in die Aussparung V1 aufgenommen ist, wobei der Randbereich LV entlang wenigstens einer ringförmigen Kontur, welche die Aussparung V1 umgibt, formschlüssig und flüssigkeitsundurchlässig an die Verkleidung V anschließt.

Der Randbereich LV ist an der Verkleidung V relativ zu der Verkleidung V fixiert, insbesondere derart fixiert ist, dass der bedienelementseitige Bereich LN mittels des flexiblen Bereichs relativ zu der Verkleidung flexibel gelagert ist.

Dadurch, dass bedienelementseitige Bereich LN relativ zu der Verkleidung V flexibel gelagert ist, sind die Schwingungen der Vibration in Bezug auf eine Dämpfung ausreichend von der Verkleidung V entkoppelt und können so vom Nutzer erfasst werden.

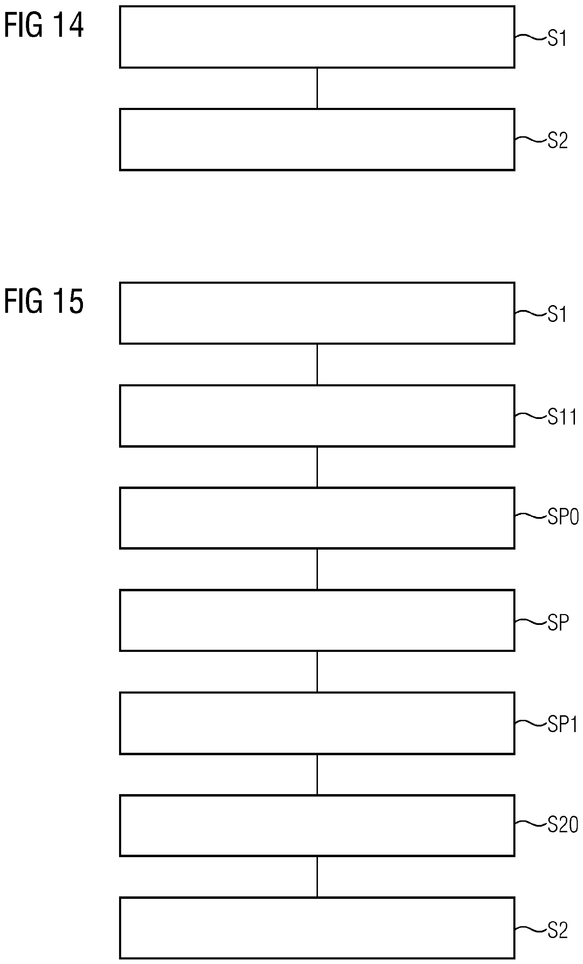

Die Fig. 14 zeigt ein Ablaufdiagramm für ein Verfahren zum Bedienen eines medizinischen Bildgebungsgeräts 2 mittels einer Vorrichtung 1,
- wobei die Vorrichtung 1 eine Bedieneinheit N, eine Verbindungseinheit L und eine Vibrationseinheit M aufweist,
- wobei die Verbindungseinheit L einen bedienelementseitigen Bereich LN, einen Randbereich LV und einen flexiblen Bereich LF aufweist, wobei der bedienelementseitige Bereich LN mittels des flexiblen Bereichs LF relativ zu dem Randbereich LV flexibel gelagert ist,
- wobei die Bedieneinheit N einen Berührungsbereich N1 aufweist, wobei der Berührungsbereich N1 relativ zu dem bedienelementseitigen Bereich LN fixiert ist,
- wobei mittels der Bedieneinheit N ein Berührungssignal basierend auf einer Berührung des Berührungsbereichs N1 generiert S1 wird,
- wobei mittels der Vibrationseinheit M eine Vibration des Berührungsbereichs N1 relativ zu dem Randbereich LV während der Berührung des Berührungsbereichs N1 angeregt S2 wird, insbesondere derart angeregt wird, dass die Vibration eine taktile Rückmeldung in Bezug auf die Berührung des Berührungsbereichs N1 umfasst, insbesondere derart umfasst, dass die taktile Rückmeldung mittels der Berührung des Berührungsbereichs N1 erfasst wird.

Die Fig. 15 zeigt ein weiteres Beispiel für ein Ablaufdiagramm für ein Verfahren zum Bedienen eines medizinischen Bildgebungsgeräts 2,
- wobei mittels der Bedieneinheit N das Berührungssignal an eine Datenverarbeitungseinheit P gesendet S11 wird,
- wobei mittels der Datenverarbeitungseinheit P das Berührungssignal empfangen SP0 wird, basierend auf dem Berührungssignal ein Vibrationssignal generiert SP wird und das Vibrationssignal an die Vibrationseinheit M gesendet SP1 wird,
- wobei mittels der Vibrationseinheit M das Vibrationssignal empfangen S20 wird und basierend auf dem Vibrationssignal die Vibration des Berührungsbereichs N1 relativ zu dem Randbereich LV anzuregen S2.

## Patentansprüche

1. Vorrichtung (1) zum Bedienen eines medizinischen Bildgebungsgeräts (2),
- wobei die Vorrichtung (1) eine Bedieneinheit (N), eine Verbindungseinheit (L) und eine Vibrationseinheit (M) aufweist,
- wobei die Verbindungseinheit (L) einen bedienelementseitigen Bereich (LN), einen Randbereich (LV) und einen flexiblen Bereich (LF) aufweist, wobei der bedienelementseitige Bereich (LN) mittels des flexiblen Bereichs (LF) relativ zu dem Randbereich (LV) flexibel gelagert ist,
- wobei die Bedieneinheit (N) einen Berührungsbereich (N1) aufweist, wobei der Berührungsbereich (N1) relativ zu dem bedienelementseitigen Bereich (LN) fixiert ist,
- wobei die Bedieneinheit (N) dazu eingerichtet ist, ein Berührungssignal basierend auf einer Berührung des Berührungsbereichs (N1) zu generieren (S1),
- wobei die Vibrationseinheit (M) dazu eingerichtet ist, eine Vibration des Berührungsbereichs (N1) relativ zu dem Randbereich (LV) während der Berührung des Berührungsbereichs (N1) anzuregen (S2),
- wobei die Vibrationseinheit (M) einen Satz von Vibrationsmotoren (M1-M8) aufweist, wobei die Vibrationsmotoren des Satzes von Vibrationsmotoren (M1-M8) derart relativ zueinander angeordnet sind, dass eine Referenzebene (RE) durch jeden Vibrationsmotor des Satzes von Vibrationsmotoren (M1-M8) verläuft,
- wobei die Vibrationsmotoren des Satzes von Vibrationsmotoren (M1-M8) derart um eine zu der Referenzebene (RE) senkrechte Referenzachse (RA) herum angeordnet sind, dass der Satz von Vibrationsmotoren (M1-M8) für jede beliebige Halbgerade (R1, R2), die von der Referenzachse (RA) ausgeht und in der Referenzebene (RE) liegt, einen entsprechenden Vibrationsmotor aufweist, sodass ein auf die Referenzachse (RA) bezogener Winkelabstand (R1M, R2M) zwischen dieser Halbgeraden (R1, R2) und dem entsprechenden Vibrationsmotor nicht größer als 60 Grad ist,
- **dadurch gekennzeichnet, dass** für jeden Vibrationsmotor des Satzes von Vibrationsmotoren (M1-M8) eine Motorachse dieses Vibrationsmotors in der Referenzebene (RE) liegt und eine Rotationsachse eines diesen Vibrationsmotor bildenden Motors mit exzentrischer rotierender Masse, um welche die exzentrische Masse rotiert, oder eine Linearachse eines diesen Vibrationsmotor bildenden linearen Resonanzaktors ist, wobei der auf die Referenzachse (RA) bezogene Winkelabstand (R1M, R2M) zwischen dieser Halbgeraden (R1, R2) und dem entsprechenden Vibrationsmotor ein auf die Referenzachse (RA) bezogener Winkelabstand zwischen dieser Halbgeraden (R1, R2) und der Motorachse des entsprechenden Vibrationsmotors ist.

2. Vorrichtung (1) nach Anspruch 1,
- wobei die Bedieneinheit (N) ferner dazu eingerichtet ist, das Berührungssignal an eine Datenverarbeitungseinheit (P) zu senden (S11),
- wobei die Datenverarbeitungseinheit (P) dazu eingerichtet ist, das Berührungssignal zu empfangen (SP0), basierend auf dem Berührungssignal ein Vibrationssignal zu generieren (SP) und das Vibrationssignal an die Vibrationseinheit (M) zu senden (SP1),
- wobei die Vibrationseinheit (M) ferner dazu eingerichtet ist, das Vibrationssignal zu empfangen (S20) und die Vibration des Berührungsbereichs (N1) relativ zu dem Randbereich (LV) basierend auf dem Vibrationssignal anzuregen (S2).

3. Vorrichtung (1) nach Anspruch 2, ferner aufweisend die Datenverarbeitungseinheit (P).

4. Vorrichtung nach einem der Ansprüche 1 bis 3,
- wobei die Berührung des Berührungsbereichs (N1) eine Eingabe eines Werts für das Bedienen des medizinischen Bildgebungsgeräts (2) betrifft,
- wobei die Vibration des Berührungsbereichs (N1) relativ zu dem Randbereich (LV) während der Berührung des Berührungsbereichs (N1) eine taktile Rückmeldung, welche den Wert für das Bedienen des medizinischen Bildgebungsgeräts (2) betrifft, umfasst.

5. Vorrichtung nach Anspruch 4,
- wobei die Vibration des Berührungsbereichs (N1) relativ zu dem Randbereich (LV) während der Berührung des Berührungsbereichs (N1) ein Trägersignal bildet,
- wobei das Trägersignal mit einem Nutzsignal, welches den Wert für das Bedienen des medizinischen Bildgebungsgeräts (2) betrifft, moduliert ist.

6. Vorrichtung nach Anspruch 4 oder 5,
- wobei ein Parameter der Vibration von dem Wert für das Bedienen des medizinischen Bildgebungsgeräts (2) monoton abhängig ist.

7. Vorrichtung nach einem der Ansprüche 1 bis 6,
- wobei der auf die Referenzachse (RA) bezogene Winkelabstand (R1M, R2M) zwischen dieser Halbgeraden (R1, R2) und dem entsprechenden Vibrationsmotor nicht größer als 45 Grad ist.

8. Vorrichtung nach einem der Ansprüche 1 bis 7,
- wobei die Verbindungseinheit (L) einen flexiblen Dichtungsring (F) aufweist,
- wobei eine erste ringförmige Kontaktfläche (F1) des flexiblen Dichtungsrings (F) relativ zu dem bedienelementseitigen Bereich (LN) fixiert ist,
- wobei eine zweite ringförmige Kontaktfläche (F2) des flexiblen Dichtungsrings (F) wenigstens einen Teilbereich des Randbereichs (LV) bildet,
- wobei ein ringförmiger Teilbereich des flexiblen Dichtungsrings (F) wenigstens einen Teilbereich des flexiblen Bereichs (LF) bildet.

9. Medizinisches Bildgebungsgerät (2), aufweisend die Vorrichtung (1) nach einem der Ansprüche 1 bis 8 und eine Verkleidung (V) zur Abgrenzung eines Innenbereichs (4) des medizinischen Bildgebungsgeräts (2) gegen eine Umgebung (5), wobei die Umgebung (5) das medizinische Bildgebungsgerät (2) umgibt,
- wobei der Randbereich (LV) an der Verkleidung (V) relativ zu der Verkleidung (V) fixiert ist.

10. Medizinisches Bildgebungsgerät (2) nach Anspruch 9,
- wobei die Verkleidung (V) eine Aussparung (V1) aufweist, in welche zumindest ein Teil der Vorrichtung (1) aufnehmbar ist,
- wobei der zumindest eine Teil der Vorrichtung (1) in die Aussparung (V1) aufgenommen ist,
- wobei der Randbereich (LV) entlang wenigstens einer ringförmigen Kontur, welche die Aussparung (V1) umgibt, formschlüssig und flüssigkeitsundurchlässig an die Verkleidung (V) anschließt.

11. Verfahren zum Bedienen eines medizinischen Bildgebungsgeräts (2) mittels der Vorrichtung (1) nach einem der Ansprüche 1 bis 8,
- wobei mittels der Bedieneinheit (N) ein Berührungssignal basierend auf einer Berührung des Berührungsbereichs (N1) generiert (S1) wird,
- wobei mittels der Vibrationseinheit (M) eine Vibration des Berührungsbereichs (N1) relativ zu dem Randbereich (LV) während der Berührung des Berührungsbereichs (N1) angeregt (S2) wird.

12. Verfahren nach Anspruch 11,
- wobei mittels der Bedieneinheit (N) das Berührungssignal an eine Datenverarbeitungseinheit (P) gesendet (S11) wird,
- wobei mittels der Datenverarbeitungseinheit (P) das Berührungssignal empfangen (SPO) wird, basierend auf dem Berührungssignal ein Vibrationssignal generiert (SP) wird und das Vibrationssignal an die Vibrationseinheit (M) gesendet (SP1) wird,
- wobei mittels der Vibrationseinheit (M) das Vibrationssignal empfangen (S20) wird und basierend auf dem Vibrationssignal die Vibration des Berührungsbereichs (N1) relativ zu dem Randbereich (LV) anzuregen (S2).

13. Verfahren nach Anspruch 11 oder 12,
- wobei die Berührung des Berührungsbereichs (N1) eine Eingabe eines Werts für das Bedienen des medizinischen Bildgebungsgeräts (2) betrifft,
- wobei die Vibration des Berührungsbereichs (N1) relativ zu dem Randbereich (LV) während der Berührung des Berührungsbereichs (N1) eine taktile Rückmeldung, welche den Wert für das Bedienen des medizinischen Bildgebungsgeräts (2) betrifft, umfasst.

14. Verfahren nach Anspruch 13,
- wobei die Vibration des Berührungsbereichs (N1) relativ zu dem Randbereich (LV) während der Berührung des Berührungsbereichs (N1) ein Trägersignal bildet,
- wobei das Trägersignal mit einem Nutzsignal, welches den Wert für das Bedienen des medizinischen Bildgebungsgeräts (2) betrifft, moduliert wird.

15. Verfahren nach Anspruch 13 oder 14,
- wobei ein Parameter der Vibration von dem Wert für das Bedienen des medizinischen Bildgebungsgeräts (2) monoton abhängig ist.

## Claims

1. Apparatus (1) for operating a medical imaging device (2),
- wherein the apparatus (1) has a control unit (N), a connection unit (L) and a vibration unit (M),
- wherein the connection unit (L) has a control-element-side region (LN), an edge region (LV) and a flexible region (LF), wherein the control-element-side region (LN) is flexibly mounted relative to the edge region (LV) by means of the flexible region (LF),
- wherein the control unit (N) has a contact region (N1), wherein the contact region (N1) is fixed relative to the control-element-side region (LN),
- wherein the control unit (N) is designed to generate (S1) a contact signal on the basis of contact with the contact region (N1),
- wherein the vibration unit (M) is designed to induce (S2) a vibration of the contact region (N1) relative to the edge region (LV) during contact with the contact region (N1),
- wherein the vibration unit (M) has a set of vibration motors (M1-M8), wherein the vibration motors in the set of vibration motors (M1-M8) are arranged relative to one another, such that a reference plane (RE) runs through each vibration motor in the set of vibration motors (M1-M8),
- wherein the vibration motors in the set of vibration motors (M1-M8) are arranged about a reference axis (RA) perpendicular to the reference plane (RE), such that the set of vibration motors (M1-M8) has a corresponding vibration motor for any half-line (R1, R2) which emanates from the reference axis (RA) and lies in the reference plane (RE), so that an angular distance (R1M, R2M) in relation to the reference axis (RA) between these half-lines (R1, R2) and the corresponding vibration motor is not greater than 60 degrees,
- **characterised in that** for each vibration motor in the set of vibration motors (M1-M8) a motor axis of that vibration motor lies in the reference plane (RE) and is an axis of rotation of a motor which forms this vibration motor with eccentric rotating mass, about which the eccentric mass rotates, or is a linear axis of a linear resonance actuator which forms this vibration motor, wherein the angular distance (R1M, R2M) in relation to the reference axis (RA) between these half-lines (R1, R2) and the corresponding vibration motor is an angular distance in relation to the reference axis (RA) between these half-lines (R1, R2) and the motor axis of the corresponding vibration motor.

2. Apparatus (1) according to claim 1,
- wherein the control unit (N) is further designed to send (S11) the contact signal to a data processing unit (P),
- wherein the data processing unit (P) is designed to receive (SPO) the contact signal, to generate (SP) a vibration signal on the basis of the contact signal and to send (SP1) the vibration signal to the vibration unit (M),
- wherein the vibration unit (M) is further designed to receive (S20) the vibration signal and to induce (S2) the vibration of the contact region (N1) relative the edge region (LV) on the basis of the vibration signal.

3. Apparatus (1) according to claim 2, further having the data processing unit (P).

4. Apparatus according to one of claims 1 to 3,
- wherein contact with the contact region (N1) relates to an input of a value for the operation of the medical imaging device (2),
- wherein the vibration of the contact region (N1) relative to the edge region (LV) during contact with the contact region (N1) comprises tactile feedback, which relates to the value for the operation of the medical imaging device (2).

5. Apparatus according to claim 4,
- wherein the vibration of the contact region (N1) relative to the edge region (LV) during contact with the contact region (N1) forms a carrier signal,
- wherein the carrier signal is modulated with a useful signal which relates to the value for the operation of the medical imaging device (2).

6. Apparatus according to claim 4 or 5,
- wherein one parameter of the vibration is a monotonic function of the value for the operation of the medical imaging device (2).

7. Apparatus according to one of claims 1 to 6,
- wherein the angular distance (R1M, R2M) in relation to the reference axis (RA) between these half-lines (R1, R2) and the corresponding vibration motor is not greater than 45 degrees.

8. Apparatus according to one of claims 1 to 7,
- wherein the connection unit (L) has a flexible sealing ring (F),
- wherein a first annular contact surface (F1) of the flexible sealing ring (F) is fixed relative to the control-element-side region (LN),
- wherein a second annular contact surface (F2) of the flexible sealing ring (F) forms at least one subregion of the edge region (LV),
- wherein an annular subregion of the flexible sealing ring (F) forms at least one subregion of the flexible region (LF).

9. Medical imaging device (2), having the apparatus (1) according to one of claims 1 to 8 and a casing (V) for delimiting an internal region (4) of the medical imaging device (2) from a surrounding area (5), wherein the surrounding area (5) surrounds the medical imaging device (1),
- wherein the edge region (LV) is fixed on the casing (V) relative to the casing (V).

10. Medical imaging device (2) according to claim 9,
- wherein the casing (V) has a recess (V1), in which at least one part of the apparatus (1) can be accommodated,
- wherein the at least one part of the apparatus (1) is accommodated in the recess (V1),
- wherein the edge region (LV) is attached to the casing (V) in a form-fit manner and in a manner that is impervious to liquids along at least one annular contour that surrounds the recess (V1).

11. Method for operating a medical imaging device (2) by means of the apparatus (1) according to one of claims 1 to 8,
- wherein a contact signal is generated (S1) by means of the control unit (N) on the basis of contact with the contact region (N1),
- wherein a vibration of the contact region (N1) relative to the edge region (LV) is induced (S2) by means of the vibration unit (M) during contact with the contact region (N1).

12. Method according to claim 11,
- wherein the contact signal is sent (S11) to a data processing unit (P) by means of the control unit (N),
- wherein the contact signal is received (SPO) by means of the data processing unit (P), a vibration signal is generated (SP) on the basis of the contact signal and the vibration signal is sent (SP1) to the vibration unit (M),
- wherein the vibration signal is received (S20) by means of the vibration unit (M) and the vibration of the contact region (N1) relative to the edge region (LV) is induced (S2) on the basis of the vibration signal.

13. Method according to claim 11 or 12,
- wherein contact with the contact region (N1) relates to an input of a value for the operation of the medical imaging device (2),
- wherein the vibration of the contact region (N1) relative to the edge region (LV) during contact with the contact region (N1) comprises tactile feedback, which relates to the value for the operation of the medical imaging device (2).

14. Method according to claim 13,
- wherein the vibration of the contact region (N1) relative to the edge region (LV) during contact with the contact region (N1) forms a carrier signal,
- wherein the carrier signal is modulated with a useful signal, which relates to the value for the operation of the medical imaging device (2).

15. Method according to claim 13 or 14,
- wherein one parameter of the vibration is a monotonic function of the value for the operation of the medical imaging device (2).

## Revendications

1. Installation (1) pour faire fonctionner un appareil (2) d'imagerie médicale,
- dans laquelle l'installation (1) comporte une unité (N) de fonctionnement, une unité (L) de liaison et une unité (M) de vibration,
- dans laquelle l'unité (L) de liaison comporte une partie (LN) du côté d'un élément de fonctionnement, une partie (LV) de bord et une partie (LF) souple, dans laquelle la partie (LN) du côté d'un élément de fonctionnement est montée souple par rapport à la partie (LV) de bord au moyen de la partie (LF) souple,
- dans laquelle l'unité (N) de fonctionnement a une partie (N1) de toucher, dans laquelle la partie (N1) de toucher est fixe par rapport à la partie (LN) du côté de l'élément de fonctionnement,
- dans laquelle l'unité (N) de fonctionnement est agencée pour produire un signal de toucher sur la base d'un toucher de la partie (N1) de toucher (S1),
- dans laquelle l'unité (M) de vibration est agencée pour exciter une vibration de la partie (N1) de toucher par rapport à la partie (LV) de bord pendant le toucher de la partie (N1) de toucher (S2),
- dans laquelle l'unité (M) de vibration comporte un ensemble de moteurs (M1-M8) de vibration, dans laquelle les moteurs de vibration de l'ensemble de moteurs (M1-M8) de vibration sont disposés relativement les uns par rapport aux autres de manière à ce qu'un plan (RE) de référence passe par chaque moteur de vibration de l'ensemble de moteurs (M1-M8) de vibration,
- dans laquelle les moteurs de vibration de l'ensemble de moteurs (M1-M8) de vibration sont disposés de telle façon, autour d'un axe (RA) de référence perpendiculaire au plan (RE) de référence, que l'ensemble de moteurs (M1-M8) de vibration a, pour toute demi-droite (R1, R2) quelconque, qui part de l'axe (RA) de référence et se trouve dans le plan (RE) de référence, un moteur de vibration correspondant, de sorte qu'une distance (R1M, R2M) angulaire, rapportée à l'axe (RA) de référence entre ces demi-droites (R1, R2) et le moteur de vibration correspondant, ne soit pas plus grande que 60 degrés,
- **caractérisée en ce que**, pour chaque moteur de vibration de l'ensemble de moteurs (M1-M8) de vibration, un axe de ce moteur de vibration se trouve dans le plan (RE) de référence et est un axe de rotation d'un moteur formant ce moteur de vibration à masse tournant de manière excentrée, autour duquel la masse excentrée tourne, ou un axe linéaire d'un actionneur de résonnance linéaire formant ce moteur de vibration, dans laquelle la distance (R1M, R2M) angulaire, rapportée à l'axe (RA) de référence, entre ces demi-droites (R1, R2) et le moteur de vibration correspondant, est une distance angulaire, rapportée à l'axe (RA) de référence, entre ces demi-droites (R1, R2) et l'axe du moteur de vibration correspondant.

2. Installation (1) suivant la revendication 1,
- dans laquelle l'unité (N) de fonctionnement est agencée en outre pour envoyer (S11) le signal de toucher à une unité (P) de traitement de données,
- dans laquelle l'unité (P) de traitement de données est agencée pour recevoir (SPO) le signal de toucher, pour produire (SP), sur la base du signal de toucher, un signal de vibration et pour envoyer (SP1) le signal de vibration à l'unité (M) de vibration,
- dans laquelle l'unité (M) de vibration est agencée en outre pour recevoir (S20) le signal de vibration et pour exciter (S2) la vibration de la partie (N1) de toucher par rapport à la partie (LV) de bord sur la base du signal de vibration.

3. Installation (1) suivant la revendication 2, comportant en outre l'unité (P) de traitement de données.

4. Installation suivant l'une des revendications 1 à 3,
- dans laquelle le toucher de la partie (N1) de toucher concerne une entrée d'une valeur pour le fonctionnement de l'appareil (2) d'imagerie médicale,
- dans laquelle la vibration de la partie (N1) de toucher par rapport à la partie (LV) de bord pendant le toucher de la partie (N1) de toucher comprend un message en retour tactile, qui concerne la valeur pour le fonctionnement de l'appareil (2) d'imagerie médicale.

5. Installation suivant la revendication 4,
- dans laquelle la vibration de la partie (N1) de toucher par rapport à la partie (LV) de bord pendant le toucher de la partie (N1) de bord, forme un signal de porteuse,
- dans laquelle le signal de porteuse est modulé par un signal utile, qui concerne la valeur pour le fonctionnement de l'appareil (2) d'imagerie médicale.

6. Installation suivant la revendication 4 ou 5,
- dans laquelle un paramètre de la vibration dépend de manière monotone de la valeur pour le fonctionnement de l'appareil (2) d'imagerie médicale.

7. Installation suivant l'une des revendications 1 à 6,
- dans laquelle la distance (R1M, R2M) angulaire, rapportée à l'axe (RA) de référence entre ces demi-droites (R1, R2) et le moteur de vibration correspondant, n'est pas plus grande que 45 degrés.

8. Installation suivant l'une des revendications 1 à 7,
- dans laquelle l'unité (L) de liaison a une bague (F) d'étanchéité souple,
- dans laquelle une première surface (F1) de contact annulaire de la bague (F) d'étanchéité souple est fixe par rapport à la partie (LN) du côté de l'élément de fonctionnement,
- dans laquelle une deuxième surface (F2) de contact annulaire de la bague (F) d'étanchéité souple forme au moins une zone partielle de la partie (LV) de bord,
- dans laquelle une zone partielle annulaire de la bague (F) d'étanchéité souple forme au moins une zone partielle de la partie (LF) souple.

9. Appareil (2) d'imagerie médicale comportant l'installation (1) suivant l'une des revendications 1 à 8 et un habillage (V) pour la délimitation d'une partie (4) intérieure de l'appareil (2) d'imagerie médicale par rapport à l'environnement (5), dans lequel l'environnement (5) entoure l'appareil (2) d'imagerie médicale,
- dans lequel la partie (LV) de bord sur l'habillage (V) est fixée par rapport à l'habillage (V).

10. Appareil (2) d'imagerie médicale suivant la revendication 9,
- dans lequel l'habillage (V) a un évidement (V1), dans lequel au moins une partie de l'installation (1) peut être logée,
- dans lequel la au moins une partie de l'installation (1) est logée dans l'évidement (V1),
- dans lequel la partie (LV) de bord se raccorde le long d'au moins un contour annulaire, qui entoure l'évidement (V1), à complémentarité de forme et avec imperméabilité au liquide à l'habillement (V).

11. Procédé pour faire fonctionner un appareil (2) d'imagerie médicale au moyen de l'installation (1) suivant l'une des revendications 1 à 8,
- dans lequel on crée, au moyen de l'unité (N) de fonctionnement, un signal de toucher sur la base d'un toucher de la partie (N1) de toucher (S1),
- dans lequel, au moyen de l'unité (M) de vibration, on excite une vibration de la partie (N1) de toucher par rapport à la partie (LV) de bord pendant le toucher de la partie (N1) de bord (S2).

12. Procédé suivant la revendication 11,
- dans lequel, au moyen de l'unité (N) de fonctionnement, on envoie le signal de toucher à une unité (P) de traitement de données (S11),
- dans lequel, au moyen de l'unité (P) de traitement de données, on reçoit (SPO) le signal de toucher, sur la base du signal (SP) de toucher, on produit signal de vibration et on envoie (SP1) le signal de vibration à l'unité (M) de vibration,
- dans lequel, au moyen de l'unité (M) de vibration, on reçoit (S20) le signal de vibration et, sur la base du signal de vibration, on excite (S2) la vibration de la partie (N1) de toucher par rapport à la partie (LV) de bord.

13. Procédé suivant la revendication 11 ou 12,
- dans lequel le toucher de la partie (N1) de toucher concerne une entrée d'une valeur pour le fonctionnement de l'appareil (2) d'imagerie médicale,
- dans lequel la vibration de la partie (N1) de toucher par rapport à la partie (LV) de bord pendant le toucher de la partie (N1) de toucher comprend un message en retour tactile, qui concerne la valeur pour le fonctionnement de l'appareil (2) d'imagerie médicale.

14. Procédé suivant la revendication 13,
- dans lequel la vibration de la partie (N1) de toucher par rapport à la partie (LV) de bord pendant le toucher de la partie (N1) de bord, forme un signal de porteuse,
- dans lequel le signal de porteuse est modulé par un signal utile, qui concerne la valeur pour le fonctionnement de l'appareil (2) d'imagerie médicale.

15. Procédé suivant la revendication 13 ou 14,
- dans lequel un paramètre de la vibration dépend de manière monotone de la valeur pour le fonctionnement de l'appareil (2) d'imagerie médicale.
